Europäisches Patentamt

(19) European Patent Office

Office européen des brevets

(11) EP 0 712 334 B1

(12) **EUROPEAN PATENT SPECIFICATION**

(45) Date of publication and mention
of the grant of the patent:
**09.04.1997 Bulletin 1997/15**

(21) Application number: **94923727.5**

(22) Date of filing: **06.07.1994**

(51) Int. Cl.$^6$: **B01J 23/68**, C07D 301/10

(86) International application number:
**PCT/EP94/02288**

(87) International publication number:
**WO 95/01837 (19.01.1995 Gazette 1995/04)**

(54) **EPOXIDATION CATALYST**

EPOXYOLIERUNGSKATALYSATOR

CATALYSEUR D'EPOXYDATION

(84) Designated Contracting States:
**AT BE CH DE DK ES FR GB GR IE IT LI LU NL PT SE**

(30) Priority: **07.07.1993 US 88476**

(43) Date of publication of application:
**22.05.1996 Bulletin 1996/21**

(73) Proprietor: **SHELL INTERNATIONALE RESEARCH MAATSCHAPPIJ B.V.**
**2596 HR Den Haag (NL)**

(72) Inventor: **EVANS, Wayne, Errol**
**Richmond, TX 77469 (US)**

(56) References cited:
**EP-A- 0 076 504      EP-A- 0 266 015**
**EP-A- 0 496 386      EP-A- 0 496 470**
**US-A- 3 959 316      US-A- 4 908 343**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

**Description**

The invention relates to silver-containing catalysts suitable for the preparation of ethylene oxide and to a process for preparing these catalysts.

Catalysts for the production of ethylene oxide from ethylene and molecular oxygen are generally supported silver catalysts. Such catalysts are typically promoted with alkali metals. The use of small amounts of the alkali metals potassium, rubidium and cesium were noted as useful promoters in supported silver catalysts in U.S. Patent No. 3,962,136, issued June 8, 1976, and U.S. Patent No. 4,010,115, issued March 1, 1977. U.S. Patent No. 4,007,135, issued February 8, 1977, broadly discloses silver catalysts for alkylene oxide production containing silver together with a promoting amount of at least one promoter selected from lithium, potassium, sodium, rubidium, cesium, copper, gold, magnesium, zinc, cadmium, strontium, calcium, niobium, tantalum, molybdenum, tungsten, chromium, vanadium, and barium. The use of other co-promoters, such as rhenium, or rhenium along with sulphur, molybdenum, tungsten and chromium is disclosed in U.S. Patent No. 4,766,105, issued August 23, 1988, and U.S. Patent No. 4,808,738, issued February 28, 1989. U.S. Patent No. 4,908,343, issued March 13, 1990, discloses a supported silver catalyst containing a mixture of a cesium salt and one or more alkali metal and alkaline earth metal salts.

US Patent No. 4,897,498, issued January 30, 1990, discloses the use of silver-based, alkali metal-promoted, supported catalysts in the epoxidation of olefins having no allylic hydrogens.

The invention relates to a catalyst suitable for the epoxidation of olefins having no allylic hydrogen, in particular ethylene, with oxygen in the vapour phase, which catalyst comprises a catalytically effective amount of silver, a promoting amount of alkali metal, a promoting amount of magnesium, a promoting amount of rhenium and optionally, a rhenium co-promoter selected from sulphur, molybdenum, tungsten, chromium and mixtures thereof supported on a carrier comprising at least 85 percent by weight of alpha alumina and from 0.001 to 2 percent by weight (expressed as the element) of magnesium in the form of an oxide.

It has been found that catalysts containing a promoting amount of magnesium supported on a magnesium-containing carrier have higher selectivity stabilities than those obtained with catalysts supported on a magnesium-containing carrier which have no additional magnesium impregnated onto the support.

Generally, in the vapour phase reaction of ethylene with oxygen to produce ethylene oxide, the ethylene is present in at least a double amount (on a molar basis) compared with oxygen, but is often much higher. Therefore, the conversion is calculated according to the mole percentage of oxygen which has been consumed in the reaction to form ethylene oxide and any oxygenated by-products. The oxygen conversion is dependent on the reaction temperature, and the reaction temperature is a measure of the activity of the catalyst employed. The value $T_{1.5}$ indicates the temperature at a production level of 1.5 percent ethylene oxide in the reactor outlet stream and the value T is expressed in °C. This temperature for any given catalyst is higher when the production level of ethylene oxide is higher. Moreover, this temperature is strongly dependent on the employed catalyst and the reaction conditions. The selectivity (to ethylene oxide) indicates the molar amount of ethylene oxide in the reaction product compared with the total molar amount of ethylene converted. In this specification, the selectivity is indicated as S1.5, which means the selectivity at 1.5 percent ethylene oxide production level. The selectivity of silver-based ethylene oxide catalysts can and will decrease over a period of time of usage. Therefore, from an economic and practical standpoint, it is not only the initial selectivity of a catalyst which is important, but also the rate at which the selectivity declines. In fact, significant improvement in lowering the decline rate of a catalyst can prove more economically attractive than a high initial selectivity. Thus, the rate at which a catalyst loses selectivity is a predominant factor influencing the efficiency of any particular catalyst, and lowering this decline rate can lead to significant savings in terms of minimizing waste of the ethylene starting material. As used herein, "selectivity" is used to refer to the selectivity of ethylene oxide catalysts when measured at an ethylene oxide production level of 1.5% at a gas hourly space velocity of approximately 6800 and when measured after the catalyst has been placed on stream for at least several days.

In general, the catalysts of the present invention are prepared by impregnating porous refractory magnesium-containing supports with silver ions or compound(s), complex(es) and/or salt(s) dissolved in a suitable solvent sufficient to cause deposition on the support of from about 1 to about 25 percent by weight, basis the weight of the total catalyst, of silver. The impregnated support is then separated from the solution and the deposited silver compound is reduced to metallic silver. Also deposited on the support either prior to, coincidentally with, or subsequent to the deposition of the silver will be suitable ions, or compound(s) and/or salt(s) of alkali metal dissolved in a suitable solvent. Also deposited on the carrier coincidentally with the deposition of the silver and/or alkali metal will be suitable magnesium compound(s), complex(es) and/or salt(s) dissolved in an appropriate solvent. Also deposited on the support either prior to, coincidentally with, or subsequent to the deposition of the silver and/or alkali metal and/or magnesium will be suitable rhenium ions or compound(s), complex(es) and/or salt(s) dissolved in an appropriate solvent. In a preferred embodiment, suitable ions or salt(s), complex(es) and/or compound(s) of sulphur, molybdenum, tungsten and/or chromium dissolved in an appropriate solvent will be deposited on the carrier either prior to, coincidentally with, or subsequent to the deposition of the silver and/or alkali metal and/or magnesium and/or rhenium.

The carrier or support employed in these catalysts may, in general terms, be prepared from high purity alpha alumina powder, a magnesium oxide-providing compound, an optional silicon oxide-providing compound, an optional zirconium oxide-providing compound and conventional binders and/or burnout agents.

The alpha alumina used in the carrier preparation generally has a purity greater than about 98%, and less than about 0.06% by weight of soda impurities. The alumina has the form of a fine powder, preferably one having an average particle size of from 0.5 to 5 $\mu$m and preferably, from 1 to 4 $\mu$m. The average particle size may be determined by measuring the maximum dimension of a number of particles and taking the average thereof. The average crystallite size is determined by measuring the maximum dimension of a number of crystallites and taking the average thereof. The alpha alumina will be present in the calcined carrier in an amount greater than about 85% and preferably about 90% by weight of the total carrier.

The magnesium compounds that may be used to prepare the carriers are oxides or compounds which are decomposable to or which form oxides upon calcination. Examples include carbonates, nitrates and carboxylates. Suitable compounds include the magnesium oxides themselves, as well as the mixed oxides such as magnesium silicates, magnesium aluminates, magnesium aluminosilicates, magnesium zirconates and the like. The preferred compounds are magnesium nitrate, magnesium oxide and magnesium silicate ($MgSiO_3$).

The amount of magnesium compound used in the carrier should be an amount which provides, in the final carrier composition, from 10 to 20,000, preferably from 100 to 1000 parts per million by weight of the total carrier, measured and expressed as the element. Stated another way, the magnesium is present in the carrier in an amount in the range of from 0.001 to 2.0, and preferably from 0.01 to 0.1 percent by weight, basis the total weight of the carrier. The amount of magnesium in the carrier is measured by total acid digestion followed by atomic absorption spectroscopy.

As used herein, the terms "magnesium-containing carrier" and "magnesium in the carrier" refer to magnesium which is insoluble or substantially insoluble in the impregnating solution. The magnesium may be added to the carrier in any manner and at any time prior to contacting the carrier with the hereinafter described impregnation solution or solutions.

The silicon component, if present, is typically used in an amount which provides, in the final carrier composition, from 0.01 to 5, preferably 0.03 to 4 percent by weight, measured as silica. The silicon compounds which may be used to prepare the carriers are oxides or compounds which are decomposable to or which form oxides upon calcination. Suitable compounds include silicon dioxide as well as the mixed oxides such as, for example, the alkaline earth metal silicates, zirconium silicates, aluminosilicates including zeolites, hydrolyzable silicon compounds, polysiloxanes and the like.

The zirconium component, if present, is preferably present in an amount that is from 0.1 to 10, and preferably from 0.3 to 5.0 percent by weight based on the total weight of the carrier. Where zirconia is generated in-situ, the amount utilized should be selected such that the final composition is within these parameters.

The zirconium compounds which may be used to prepare the carriers are oxides or compounds which are decomposable to or which form oxides upon calcination. Examples include carbonates, nitrates and carboxylates. Suitable compounds include zirconium nitrate, zirconium dioxide, as well as the mixed oxides such as zirconium silicates, zirconium aluminosilicates, zirconates and the like. In a preferred embodiment, the zirconium compound is zirconium dioxide.

In a preferred embodiment, the carrier comprises at least 85 percent by weight of alpha alumina, from 0.001 to 2 percent by weight (expressed as the element) of magnesium in the form of an oxide, from 0.01 to 5 percent by weight (measured as the dioxide) of silicon in the form of an oxide and from 0.1 to 10 percent by weight (measured as the dioxide) of zirconium in the form of an oxide.

Preferred carrier compositions comprise the magnesium- and the silicon-containing compounds in the form of a single compound, a magnesium silicate, which may be added as an original component or generated in-situ by the reaction of silica or silica-generating compounds with compounds that decompose to magnesium oxide upon heating, with the amount of the oxide formed being in stoichiometric equivalent to or in excess of the amount of silica.

The preferred carrier can be prepared by mixing a powdered alpha alumina, magnesium silicate and zirconia with water and a binder and/or burnout material to prepare a mixture which is then extruded and calcined at a temperature ranging from about 1350 °C to about 1500°C.

The alpha alumina powder is most preferably combined with magnesium silicate itself but, as indicated above, it is also possible to use a magnesium oxide-generating compound and silica or a silica-generating compound in such proportions that upon heating, magnesium silicate is produced. These components are mixed with zirconia or a zirconia-generating compound, if present, a burnout/binding agent and water, and thereafter formed into shapes and calcined.

The calcined carriers and catalysts made therefrom will typically have water pore volumes as measured by conventional water absorption techniques ranging from 0.1 to 0.6 ml/g by volume, preferably from 0.3 to 0.5 ml/g, and surface areas as measured by the B.E.T. method ranging from 0.1 $m^2$/g to 3 $m^2$/g, preferably from 0.1 $m^2$/g to 2 $m^2$/g. The B.E.T. method for determining specific surface area is described in detail in Brunauer, S., Emmet, P. Y. and Teller, E., J. Am. Chem. Soc., 60, 309-16 (1938).

The carrier is preferably shaped into particles, chunks, pieces, pellets, rings, spheres, wagon wheels, and the like of a size suitable for use in fixed bed reactors. Conventional commercial fixed bed reactors are typically in the form of a plurality of parallel elongated tubes (in a suitable shell) approximately 18 to 68 mm O.D. and 8 to 63 mm I.D. and 45-14 m long filled with catalyst. In such reactors, it is desirable to use a support formed into a rounded shape, such as, for example, spheres, pellets, rings, tablets and the like, having diameters from 2 to 20 mm.

Particular supports having differing properties such as surface area and pore volume may be selected in order to provide particular catalytic properties. With regard to surface area (B.E.T.), a possible lower limit is 0.01 $m^2$/g and a possible upper limit is 10 $m^2$/g. With regard to water pore volume, a possible lower limit is 0.05 ml/g and a possible upper limit is 0.8 ml/g.

The catalysts of the present invention are prepared by a technique in which the alkali metal promoters, the magnesium, the rhenium, and the rhenium co-promoter, if present, in the form of soluble salts and/or compounds are deposited on the catalyst and/or support prior to, simultaneously with, or subsequent to the deposition of the silver and each other. The alkali metals may be deposited at one step of the process and the magnesium, rhenium and/or the rhenium co-promoter, if present, at a different step or steps. The preferred method is to deposit silver, alkali metal, magnesium, rhenium and rhenium co-promoter simultaneously on the support, that is, in a single impregnation step, although it is believed that the individual or concurrent deposition of the alkali metal, magnesium, rhenium and rhenium co-promoter, if present, prior to and/or subsequent to the deposition of the silver would also produce suitable catalysts.

For convenience, the amounts of all metals present in the catalyst are expressed as the metal, irrespective of the form in which they are present, which generally is believed to be as oxidic compounds.

Promoting amounts of alkali metal or mixtures of alkali metal are deposited on the support using a suitable solution. Although alkali metals exist in a pure metallic state, they are not suitable for use in that form. They are used as ions or compounds of alkali metals dissolved in a suitable solvent for impregnation purposes. The carrier is impregnated with a solution of alkali metal promoter ions, salt(s) and/or compound(s) before, during or after impregnation of the silver ions or salt(s), complex(es), and/or compound(s) has taken place. An alkali metal promoter may even be deposited on the carrier after reduction to metallic silver has taken place. The promoting amount of alkali metal utilized will depend on several variables, such as, for example, the surface area and pore structure and surface chemical properties of the carrier used, the silver content of the catalyst and the particular ions used in conjunction with the alkali metal cation, magnesium or rhenium or rhenium co-promoter, if present, and the amounts of magnesium, rhenium and rhenium co-promoter, if any, present. The amount of alkali metal promoter deposited upon the support or present on the catalyst generally lies between 10 and 3000, preferably between 15 and 2000 and more preferably, between 20 and 1500 parts per million by weight of total catalyst. Most preferably, the amount ranges between 50 and 1000 parts per million by weight of the total catalyst. A preferred alkali metal promoter is cesium. A particularly preferred alkali metal promoter is cesium plus at least one additional alkali metal. The additional alkali metal is preferably selected from sodium, lithium and mixtures thereof, with lithium being preferred.

It should be understood that the amounts of alkali metal promoters on the catalysts are not necessarily the total amounts of these metals present in the catalyst. Rather, they are the amounts of alkali metal promoters which have been added to the catalyst by impregnation with a suitable solution of ions, salts and/or compounds and/or complexes of alkali metals. These amounts do not include amounts of alkali metals which are locked into the support, for example, by calcining, or are not extractable in a suitable solvent such as water or lower alkanol or amine or mixtures thereof and do not provide a promoting effect. It is also understood that a source of the alkali metal promoter ions, salts and/or compounds used to promote the catalyst may be the carrier. That is, the carrier may contain extractable amounts of alkali metal that can be extracted with a suitable solvent, such as water or lower alkanol, thus preparing an impregnating solution from which the alkali metal ions, salts and/or compounds are deposited or redeposited on the support.

Promoting amounts of magnesium compounds or mixtures of magnesium compounds are also deposited on the carrier. Although magnesium does exist in a pure metallic state, it is not suitable for use in that form. The magnesium is used as an ion or compound of magnesium dissolved in a suitable solvent for impregnation purposes. The carrier is impregnated with a solution of magnesium promoter ions, salt(s) and/or compound(s) before, during or after impregnation of the silver ions or salt(s), complex(es), and/or compound(s) has taken place. A magnesium promoter may even be deposited on the carrier after reduction to metallic silver has taken place. The promoting amount of magnesium utilized will depend on several variables, such as, for example, the surface area and pore structure and surface chemical properties of the carrier used, the silver content of the catalyst and the particular ions used in conjunction with the alkali metal cation, magnesium or rhenium or rhenium co-promoter, if present, and amounts of magnesium, rhenium and rhenium co-promoter, if any, present. The amount of magnesium promoter deposited upon the support generally lies between 10 and 1000, and preferably between 15 and 200 parts per million by weight of the total catalyst. Most preferably, the amount ranges between 25 and 75 parts per million by weight of the total catalyst.

In a preferred embodiment, the magnesium compound is selected from the group consisting of magnesium nitrate, magnesium acetate or other carboxylic acid salts, the magnesium halides, the magnesium oxyhalides, magnesium carbonate, magnesium sulphate and mixtures thereof. Particularly preferred magnesium compounds are magnesium nitrate and magnesium acetate.

The carrier is also impregnated with rhenium ions, salt(s), compound(s), and/or complex(es). The preferred amount of rhenium, calculated as the metal, deposited on or present on the carrier or catalyst ranges from 0.1 to 10, more preferably from 0.2 to 5 micromoles per gram of total catalyst, or, alternatively stated, from 19 to 1860, preferably from 37 to 930 parts per million by weight of total catalyst.

The rhenium compounds used in the preparation of the instant catalysts are rhenium compounds that can be solubilized in an appropriate solvent. Preferably, the solvent is a water-containing solvent. More preferably, the solvent is the same solvent used to deposit the silver and the alkali metal promoter. Examples of suitable rhenium compounds include the rhenium salts such as rhenium halides, the rhenium oxyhalides, the rhenates, the perrhenates, the oxides and the acids of rhenium. A preferred compound for use in the impregnation solution is the perrhenate, preferably ammonium perrhenate. However, the alkali metal perrhenates, alkaline earth metal perrhenates, silver perrhenates, other perrhenates and rhenium heptoxide can also be suitably utilized. Rhenium heptoxide, $Re_2O_7$, when dissolved in water, hydrolyzes to perrhenic acid, $HReO_4$, or hydrogen perrhenate. Thus, for purposes of this specification, rhenium heptoxide can be considered to be a perrhenate, i.e., $ReO_4$. It is also understood that there are many rhenium compounds that are not soluble per se in water. However, these compounds can be solubilized by utilizing various acids, bases, peroxides, alcohols, and the like. After solubilization, these compounds could be used, for example, with an appropriate amount of water or other suitable solvent to impregnate the carriers. Of course, it is also understood that upon solubilization of many of these compounds, the original compound no longer exists after solubilization. For example, rhenium metal is not soluble in water. However, it is soluble in concentrated nitric acid as well as in hydrogen peroxide solution. Thus, by using an appropriate reactive solvent, one could use rhenium metal to prepare a solubilized rhenium-containing impregnating solution. In a preferred embodiment of the instant invention, the rhenium present on the catalyst is present in a form that is extractable in a dilute aqueous base solution.

It was found in U.S. Patent No. 4,766,105, that if a rhenium co-promoter is added to an alkali metal/rhenium doped supported silver catalyst, an improvement in initial selectivity is obtained. While suitable catalysts can be prepared in the absence of a rhenium co-promoter, it is preferable that the catalyst in the present invention contain a rhenium co-promoter. When a co-promoter is utilized, the co-promoter is a selected from the group consisting of sulphur, molybdenum, tungsten, chromium and mixtures thereof, preferably compounds of these elements, and mixtures thereof. The exact form of the co-promoter on the catalyst is not known. The co-promoter, it is believed, is not present on the catalyst in the elemental form since the co-promoter is applied to catalyst in the form of ions, salts, compounds and/or complexes and the reducing conditions generally used to reduce the silver to metallic silver are not usually sufficient to reduce the sulphur, molybdenum, tungsten or chromium to the elemental form. It is believed that the co-promoter deposited on the support or present on the catalyst is in the compound form, and probably in the form of an oxygen-containing or oxidic compound. In a presently preferred embodiment, the co-promoter is applied to the catalyst in the oxyanionic form, i.e, in the form of a negative ion which contains oxygen. Examples of anions of sulphur that can be suitably applied include sulphate, sulphite, bisulphate, bisulphate, sulfonate, persulphate, thiosulphate, dithionate, etc. Preferred compounds to be applied are ammonium sulphate and the alkali metal sulphates. Examples of anions of molybdenum, tungsten and chromium that can be suitably applied include molybdate, dimolybdate, paramolybdate, other iso- and hetero-polymolybdates, etc.; tungstate, paratungstate, metatungstate, other iso- and hetero-polytungstates, etc.; and chromate, dichromate, chromite, halochromate, etc. Preferred are sulphates, molybdates, tungstates and chromates. The anions can be supplied with various counter-ions. Preferred are ammonium, alkali metal and hydrogen (i.e. acid form). The anions can be prepared by the reactive dissolution of various non-anionic materials such as the oxides such as $SO_2$, $SO_3$, $MoO_3$, $WO_3$, $Cr_2O_3$, etc., as well as other materials such as halides, oxyhalides, hydroxyhalides, hydroxides, sulphides, etc., of the metals.

When a co-promoter is used, the carrier is impregnated with rhenium co-promoter ions, salt(s), compound(s) and/or complex(es).

The preferred amount of co-promoter present on or deposited on the support or catalyst ranges from 0.1 to 10, preferably from 0.2 to 5 micromoles, expressed as the element, per gram of total catalyst.

The co-promoter compounds, salts and/or complexes suitable for use in the preparation of the instant catalysts are compounds, salts and/or complexes which can be solubilized in an appropriate solvent. Preferably, the solvent is a water-containing solvent. More preferably, the solvent is the same solvent used to deposit the silver, alkali metal promoter and rhenium. Preferred co-promoter compounds are the oxyanionic compounds of the co-promoter elements, preferably the ammonium and alkali metal oxyanionates, such as ammonium sulphate, potassium sulphate, cesium chromate, rubidium tungstate, ammonium molybdate, lithium sulphate, sodium tungstate, lithium chromate and the like.

Generally, the carrier is contacted with a silver salt, a silver compound, or a silver complex which has been dissolved in an aqueous solution, so that the carrier is impregnated with said aqueous solution; thereafter the impregnated carrier is separated form the aqueous solution, e.g., by centrifugation or filtration and then dried. The thus obtained impregnated carrier is heated to reduce the silver to metallic silver. It is conveniently heated to a temperature in the range of from 50°C to 600°C, during a period sufficient to cause deduction of the silver salt, compound or complex to metallic silver and to form a layer of finely divided silver, which is bound to the surface of the carrier, both the exterior and pore surface. Air, or other oxidizing gas, reducing gas, an inert gas or mixtures thereof may be conducted over the carrier during this heating step.

One method of preparing the silver containing catalyst can be found in U.S. Patent 3,702,259. Other methods for preparing the silver-containing catalysts which in addition contain higher alkali metal promoters can be found in U.S. Patent 4,010,115, U.S. Patent 4,356,312, U.S. Patent 3,962,136 and U.S. Patent 4,012,425. Methods for preparing silver-containing catalysts containing higher alkali metal and rhenium promoters can be found in U.S. Patent No. 4,761,394, and methods for silver-containing catalysts containing higher alkali metal and rhenium promoters and a rhenium co-promoter can be found in U.S. Patent No. 4,766,105.

There are known excellent methods of applying the promoters coincidentally with the silver on the carrier. Suitable alkali metal salts are generally those which are soluble in the silver-impregnating liquid phase. Besides the above-mentioned compounds may be mentioned the nitrites; the halides, such as fluorides, chlorides, iodides, bromides; oxyhalides; bicarbonates; borates; sulphates; sulphites; bisulphates; acetates; tartrates; lactates and isopropoxides, etc. The use of alkali metal, magnesium, rhenium or co-promoter salts which have ions which react with the silver salt in solution is preferably avoided, e.g. the use of cesium chloride together with silver nitrate in an aqueous solution, since then some silver chloride is prematurely precipitated. Here the use of cesium nitrate is recommended instead of cesium chloride, for example. However, cesium chloride may be used together with a silver salt-amine-complex in aqueous solution, since then the silver chloride is not precipitated prematurely from the solution.

The promoters may be deposited on the carrier (support) or on the catalyst, depending upon the particular impregnation technique or sequence utilized. In this specification and claims, the term "on the catalyst" when referring to the deposition or presence of promoters and/or co-promoters refers to the catalyst which comprises the combination of carrier (support) and silver. Thus, the promoters, i.e., alkali metal, magnesium, rhenium and rhenium co-promoter may be found individually or in a mixture thereof on the catalyst, on the support or on both the catalyst and the support. There may be, for example, alkali, magnesium, rhenium and rhenium co-promoter on the support; alkali metal, magnesium, rhenium and rhenium co-promoter on the catalyst; alkali metal, magnesium, and rhenium on the support and rhenium co-promoter on the catalyst; alkali metal, magnesium, and rhenium co-promoter on the support and rhenium on the catalyst; alkali metal, magnesium, rhenium and rhenium co-promoter on the support and rhenium and rhenium co-promoter on the catalyst and any of the other possible distributions of alkali metal, magnesium, rhenium and/or rhenium co-promoter between the support and/or the catalyst.

The amount of the alkali metal and/or magnesium and/or rhenium promoters and/or rhenium co-promoters on the porous carrier or catalyst may also be regulated within certain limits by washing out the surplus of promoter material with an appropriate solvent, for example, methanol or ethanol.

In general terms, the impregnation process comprises impregnating the support with one or more solutions comprising silver, alkali metal, magnesium, rhenium and rhenium co-promoter. The concentration of the silver (expressed as the metal) in the silver-containing solution will range from 1 g/l up to the solubility limit when a single impregnation is utilized. The concentration of the alkali metal (expressed as the metal) will range from $1 \times 10^{-3}$ g/l up to 12 g/l and preferably, from $10 \times 10^{-3}$ g/l to 12 g/l when a single impregnation step is utilized. The concentration of the magnesium (expressed as the element) will range from 0.04 g/l up to 4 g/l and preferably, from 0.06 g/l to 0.8 g/l when a single impregnation step is utilized. The concentration of the rhenium (expressed as the metal) will range from $5 \times 10^{-3}$ g/l to 20 g/l and preferably from $50 \times 10^{-3}$ g/l to 20 g/l when a single impregnation step is utilized. The concentration of rhenium co-promoter (expressed as the element) will range from $1 \times 10^{-3}$ g/l to 20 g/l and preferably from $10 \times 10^{-3}$ g/l to 20 g/l when a single impregnation step is utilized. Concentrations selected within the above noted ranges will depend upon the pore volume of the catalyst, the final amount desired in the final catalyst and whether the impregnation is single or multiple.

The silver catalysts according to the present invention have been shown to have a particularly high selectivity stability for ethylene oxide production in the direct oxidation of ethylene with molecular oxygen to ethylene oxide. The conditions for carrying out such an oxidation reaction in the presence of the silver catalysts according to the present invention broadly comprise those already described in the prior art. This applies, for example, to suitable temperatures, pressures, residence times, diluent materials such as nitrogen, carbon dioxide, steam, argon, methane or other saturated hydrocarbons, to the presence of moderating agents to control the catalytic action, for example, 1-2-dichloroethane, vinyl chloride, ethyl chloride or chlorinated polyphenyl compounds, to the desirability of employing recycle operations or applying successive conversations in different reactors to increase the yields of ethylene oxide, and to any other special conditions which may be selected in processes for preparing ethylene oxide. Pressures in the range of from atmospheric to about 3500 kPa are generally employed. Higher pressures, however, are not excluded. Molecular oxygen employed as reactant can be obtained from conventional sources. The suitable oxygen charge may consist essentially or relatively pure oxygen, a concentrated oxygen stream comprising oxygen in major amount with lesser amounts of one or more diluents, such as nitrogen and argon, or another oxygen-containing stream, such as air. It is therefore evident that the use of the present silver catalysts in ethylene oxide reactions is in no way limited to the use of specific conditions among those which are known to be effective. For purposes of illustration only, the following table shows the range of conditions that are often used in current commercial ethylene oxide reactor units.

TABLE I

| *GHSV | 1500-10,000 |
|---|---|
| Inlet Pressure | 1000-3800 KPa |
| Inlet Feed | |
| Ethylene | 1-40% |
| $O_2$ | 3-12% |
| Ethane | 0-3% |
| Argon and/or methane and/or nitrogen diluent chlorohydrocarbon moderator | 0.3-20 ppmv total |
| Coolant temperature | 180-315°C |
| Catalyst temperature | 180-325°C |
| $O_2$ conversion level | 10-60% |
| EO Production (Work Rate) | 32-320 kg EO/m$^3$ catalyst/hr. |

*Volume units of gas at standard temperature and pressure passing over one volume unit of packed catalyst per hour.

In a preferred application of the silver catalysts according to the present invention, ethylene oxide is produced when an oxygen-containing gas is contacted with ethylene in the presence of the present catalysts at a temperature in the range of from 180°C to 330°C and preferably 200°C to 325°C.

While the catalysts of the present invention are preferably used to convert ethylene to ethylene oxide, they can be also used to epoxidise other olefins having no allylic hydrogens, such as are broadly defined in U.S. Patent No. 4,897,498. Exemplary such olefins are butadiene, tertiary butyl ethylene, vinyl furan, methyl vinyl ketone, N-vinyl pyrrolidone, and the like. A presently preferred olefin for use in the practice of this process is butadiene, because of its ready availablility, relative low cost, and the wide range of possible uses for the epoxide reaction product. U.S. Patent No. 5,081,096, issued January 14, 1992, discloses a silver-based, alkali metal-promoted, supported catalyst which is adapted to the epoxidation of butadiene by treating the pro-catalyst, after its impregnation with a silver compound, with a hydrogen containing gas at a temperature not exceeding 350 °C. The same can be done with the catalysts according to the present invention.

The invention will be illustrated by the following illustrative embodiments.

Illustrative Embodiments

Illustrative Embodiment 1

The following illustrative embodiment describes typical preparative techniques for making the catalysts of the instant invention (and comparative catalysts) and the typical technique for measuring the properties of these catalysts.

Part A: Preparation of stock silver oxalate/ethylene-diamine solution for use in catalyst preparation:

1) Dissolve 415 grams (g) of reagent-grade sodium hydroxide in 2340 millilitres (ml) deionized water. Adjust the temperature to 50°C.
2) Dissolve 1699 g of "Spectropure" (high purity) silver nitrate in 2100 ml deionized water. Adjust the temperature to 50°C.
3) Add sodium hydroxide solution slowly to silver nitrate solution with stirring while maintaining a temperature of 50°C. Stir for 15 minutes after addition is complete, and then lower the temperature to 40°C.
4) Insert clean filter wands and withdraw as much water as possible from the precipitate created in step (3) in order to remove sodium and nitrate ions. Measure the conductivity of the water removed and add back as much fresh deionized water as was removed by the filter wands. Stir for 15 minutes at 40°C. Repeat this process until the conductivity of the water removed is less than 90 µmho/cm. Then add back 1500 ml deionized water.
5) Add 630 g of high-purity oxalic acid dihydrate in approximately 100 g increments. Keep the temperature at 40°C and stir to mix thoroughly. Add the last portion of oxalic acid dihydrate slowly and monitor pH to ensure that pH does not drop below 7.8.

6) Remove as much water from the mixture as possible using clean filter wands in order to form a highly concentrated silver-containing slurry. Cool the silver oxalate slurry to 30°C.

7) Add 699 g of 92 percent weight (%w) ethylenediamine (8% deionized water). Do not allow the temperature to exceed 30°C during addition.

The above procedure yields a solution containing approximately 27-33%w silver.

Part B: Preparation of impregnation solutions

For Catalyst A (Mg/Re); into a 10 ml beaker is added 0.2 g of $NH_4ReO_4$ and approximately 2 g of ethylenediamine/$H_2O$ (50/50 by weight), and the mixture is allowed to dissolve with stirring. 0.08 G of $Li_2SO_4.H_2O$ is dissolved in 1 ml of water in a weighing dish, and then added to the perrhenate solution. 0.3 g of $LiNO_3$ is dissolved in 2 ml of water and added to the perrhenate solution. The perrhenate/lithium sulphate/lithium nitrate solution is allowed to stir, ensuring complete dissolution. Separately, 0.2 g of $Mg(NO_3)_2.6H_2O$ is dissolved in 3 ml of water. Both dopant solutions are then added to 190 g of the above-prepared silver solution (specific gravity = 1.556 g/ml), and the resulting solution is diluted with water to a total weight of 205 g. One-fourth of this solution is used to prepare a catalyst. 0.05 G of CsOH is added to a 50 g portion of the silver oxalate/dopant solution to prepare the final impregnation solution.

For Catalyst B (Re only), the procedure for Catalyst A is followed, except that no magnesium was impregnated on the catalyst.

Part C: Catalyst impregnation and curing

A catalyst carrier having the properties described below is used in the following examples and illustrative embodiments unless otherwise stated:

TABLE II

| Properties of Carrier | |
| --- | --- |
| Zirconia | 1.0% |
| Magnesium Silicate | 0.78% |
| Alpha Alumina | balance |
| Water Absorption | 38% |
| Crush Strength | 6.8 kg |
| Surface Area | 0.7 m2/g |
| Total Pore Volume (Hg) | 0.4 ml/g |
| Median Pore Diameter | 4.3 $\mu$m |

Approximately 30 g of the carrier are placed under 25 mm vacuum for 3 minutes at room temperature. Approximately 50 g of doped impregnating solution is then introduced to submerge the carrier, and the vacuum is maintained at 25 mm for an additional 3 minutes. At the end of this time, the vacuum is released, and excess impregnating solution is removed from the carrier by centrifugation for 2 minutes at 500 rpm. If the impregnating solution is prepared without monoethanolamine, then the impregnated carrier is then cured by being continuously shaken in a 8500 litre/hr air stream flowing across a cross-sectional area of approximately 3-5 square inches at 250-270°C for 5-6 minutes. If significant mono-ethanolamine is present in the impregnating solution, then the impregnated carrier is cured by being continuously shaken in a 8500 litre/hr air stream at 250°C for 2.5 minutes, followed by a 2800 litre/hr air stream at 270°C for 7.5 minutes (all over a cross-section area of approximately 7.6-12.7 cm$^2$). The cured catalyst is then ready for testing.

This procedure will yield catalysts on this carrier which contain approximately 13.5%w Ag with the following approximate dopant levels (expressed in parts per million by weight basis the weight of the total catalyst, i.e., PPMW) and which are approximately optimum in cesium for the given silver and rhenium and sulphur levels and support with regard to initial selectivity under the test conditions described below.

|            | Cs, ppmw | Magnesium, ppmw | Re, ppmw | S, ppmw |
|------------|----------|-----------------|----------|---------|
| Catalyst A | 460      | 50              | 280      | 48      |
| Catalyst B | 460      | None            | 280      | 48      |

The actual silver content of the catalyst can be determined by any of a number of standard, published procedures. The actual level of rhenium on the catalysts prepared by the above process can be determined by extraction with 20 mm aqueous sodium hydroxide, followed by spectrophotometric determination of the rhenium in the extract. The actual level of magnesium on the catalyst can be determined by standard atomic emission spectroscopy. The actual level of cesium on the catalyst can be determined by employing a stock cesium hydroxide solution, which has been labelled with a radioactive isotope of cesium, in catalyst preparation. The cesium content of the catalyst can then be determined by measuring the radioactivity of the catalyst. Alternatively, the cesium content of the catalyst can be determined by leaching the catalyst with boiling deionized water. In this extraction process cesium, as well as other alkali metals, is measured by extraction from the catalyst by boiling 10 grams of whole catalyst in 20 millilitres of water for 5 minutes, repeating the above two more times, combining the above extractions and determining the amount of alkali metal present by comparison to standard solutions of reference alkali metals using atomic absorption spectroscopy (using Varian Techtron Model 1200 or equivalent).

Part D: Standard Microreactor Catalyst Test Conditions/Procedure

1.5 to 2G of crushed catalyst of 0.841-0.595 mm (20-30 mesh) are loaded into a 6.4 mm diameter stainless steel U-shaped tube. The U tube is immersed in a molten metal bath (heat medium) and the ends are connected to a gas flow system. The weight of the catalyst used and the inlet gas flow rate are adjusted to achieve a gas hourly space velocity of 6800 ml of gas per ml of catalyst per hour. The inlet gas pressure is 1550 kPa.

The gas mixture passed thorough the catalyst bed (in once-through operation) during the entire test run (including start-up) consists of 30% ethylene, 7.0% oxygen, 5% carbon dioxide, 56.0% nitrogen, and 1.0 to 6.0 ppmv ethyl chloride.

The initial reactor (heat medium) temperature is 225°C. After 1 hour at this initial temperature, the temperature is increased to 235°C for 1 hour, and then adjusted to 245°C for 1 hour. The temperature is then adjusted so as to achieve an ethylene oxide production level of 1.5%. Performance data at this production level are usually obtained when the catalyst has been onstream for a total of at least 2-3 days. Due to slight differences in feed gas composition, gas flow rates, and the calibration of analytical instruments used to determine the feed and product gas compositions, the measured selectivity and activity of a given catalyst may vary slightly from one test run to the next. To allow meaningful comparison of the performance of catalysts tested at different times, the catalysts described in this and the following illustrative embodiments were tested simultaneously with a reference catalyst. All performance data reported in this and the following illustrative embodiments are corrected and stated relative to the average initial performance of the reference catalyst ($S_{1.5}$ = 81.7%).

After obtaining initial performance values for selectivity at 1.5% ethylene oxide production the catalysts are subjected to high severity ageing conditions. Under these conditions, the catalyst ethylene oxide production level is elevated in order to accelerate degradation, i.e., ageing, of the catalyst. After a short period of accelerated ageing, the catalyst is again brought to 1.5% ethylene oxide production and reoptimized with respect to chloride under standard conditions. The selectivity is again measured, and compared to the original value of the fresh catalyst. After the new selectivity value is determined, this cycle is repeated, and the selectivity decline of the catalyst is continuously measured under standard 1.5% ethylene oxide production conditions relative to its fresh initial performance. The results are presented below in Table III. All selectivity values are expressed as %. The initial performances of Catalysts A and B were determined to be the same, within experimental error. Initial S1.5 values of 89.6% ± 0.3% were obtained.

$$\text{Loss in Selectivity (\%)} = \{ S_{1.5}, \% \text{ (Aged)} \} - \{ S_{1.5}, \% \text{ (Fresh)} \}$$

TABLE III

| Loss of Selectivity from Fresh Catalyst[1] Cumulative Ethylene Oxide Production (kg/litre) (Data Obtained at 1.5% Ethylene Oxide Production Level) | | | | | |
|---|---|---|---|---|---|
| Catalyst | 0 | 81.5 kg/litre | 163 kg/litre | 244.5 kg/litre | 326 kg/litre |
| A (Re/Mg) | 0% | 0.2% | 0.8% | 1.5% | 2.2% |
| B (Re) | 0% | 0.9% | 1.6% | 3.2% | 5.7% |

[1] This test was carried out at conditions which are much more severe than would be used in commercial operation in order to accelerate the degradation of the catalyst.

As mentioned previously, selectivity decline is of tremendous economic importance when choosing a catalyst, and retarding this decline rate can lead to significant savings in costs. As can be seen from Table III, Catalyst B (magnesium-containing carrier with no magnesium added by impregnation) decreases in selectivity much more rapidly than does Catalyst A which contains a promoting amount of magnesium impregnated on a magnesium-containing carrier. Thus, catalysts which contain both magnesium and rhenium supported on a magnesium-containing carrier maintain their selectivity significantly longer than catalysts which contain rhenium but no additional magnesium on a magnesium-supported carrier.

## Claims

1. A catalyst suitable for the epoxidation of olefins having no allylic hydrogen, in particular ethylene, with oxygen in the vapour phase, comprising a catalytically effective amount of silver, a promoting amount of alkali metal, a promoting amount of magnesium and a promoting amount of rhenium supported on a carrier comprising at least 85 percent by weight of alpha alumina and from 0.001 to 2 percent by weight (measured as the element) of magnesium in the form of an oxide.

2. The catalyst of claim 1 wherein the carrier additionally comprises from 0.01 to 5 percent by weight (measured as the dioxide) of silicon in the form of an oxide and from 0.1 to 10 percent by weight (measured as the dioxide) of zirconium in the form of an oxide.

3. The catalyst of claim 1 wherein the carrier has a water pore volume between 0.1 cc/g and 0.6 cc/g and a surface area between 0.1 $m^2$/g and to 3 $m^2$/g.

4. The catalyst of claim 1 wherein, in the carrier, the magnesium oxide is present in an amount ranging from about 0.01 percent by weight to about 0.1 percent by weight, basis the total weight of the carrier.

5. The catalyst of claim 1 wherein the amount of silver is in the range of from 1 to 25 percent by weight of the total catalyst, the amount of alkali metal promoter is in the range of from 10 to 1500 parts per million, expressed as the metal, by weight of the total catalyst, the amount of magnesium promoter is in the range of from 10 to 1000 parts per million, expressed as the element by weight of the total catalyst and the amount of rhenium promoter is in the range of from 0.1 to 10 micromoles of rhenium, expressed as the metal, per gram of total catalyst.

6. The catalyst of claim 1 wherein the alkali metal, magnesium and rhenium are found individually or in any mixture thereof on the catalyst, on the support or on both the catalyst and the support.

7. The catalyst of claim 1 wherein said alkali metal promoter is selected from potassium, rubidium, cesium, lithium and mixtures thereof.

8. The catalyst of claim 7 wherein said alkali metal promoter is cesium.

9. The catalyst of claim 7 wherein said alkali metal promoter comprises cesium plus at least one additional alkali metal.

**10.** The catalyst of any one of claims 1-9 additionally comprising a rhenium co-promoter selected from sulphur, molybdenum, tungsten, chromium and mixtures thereof.

**11.** The catalyst of claim 1 wherein the rhenium co-promoter is selected from sulphate, sulphite, sulfonate, molybdate, tungstate, chromate and mixtures thereof.

**12.** A process for preparing the catalyst of any one of claims 1-11 which comprises impregnating a carrier comprising at least 85 percent by weight of alpha alumina and from 0.001 to 2 percent by weight (measured as the element) of magnesium in the form of an oxide, with one or more solutions comprising solvent having silver compound(s) dissolved therein, alkali metal compound(s) dissolved therein, magnesium compound(s) dissolved therein and rhenium compound(s) dissolved therein sufficient to deposit on the support from 1 to 25 percent by weight of the total catalyst of the silver compound(s), expressed as the metal, from 10 to 3000 parts per million by weight of alkali metal compound(s), expressed as the metal, by weight of the total catalyst, from 10 to 1000 parts per million by weight of the total catalyst of magnesium compounds), expressed as the element, and from 0.1 to 10 micromoles per gram of total catalyst of rhenium compound(s), expressed as the metal, to provide the catalyst with a catalytically effective amount of silver, a promoting amount of alkali metal, a promoting amount of magnesium and a promoting amount of rhenium.

**13.** The process of claim 12 wherein the carrier additionally comprises from 0.01 to 5 percent by weight (measured as the dioxide) of silicon in the form of an oxide and from 0.1 to 10 percent by weight (measured as the dioxide) of zirconium in the form of an oxide.

**14.** The process of claim 12 or 13 wherein after impregnation, the silver is reduced to metallic silver by heating at a temperature between 50°C to 600°C.

**15.** The process of any one of claims 12-14, additionally comprising the impregnation of the carrier with a solution sufficient to deposit thereon from 0.1 to 10 micromoles per gram of total catalyst of rhenium co-promoter compound(s), selected from the group consisting of sulphur, tungsten, molybdenum, chromium and mixtures thereof, expressed as the element, to provide the catalyst with a catalytically effective amount of silver, a promoting amount of alkali metal, a promoting amount of magnesium, a promoting amount of rhenium and a promoting amount of rhenium co-promoter.

**Patentansprüche**

**1.** Zur Epoxidation von keinen Allylwasserstoff enthaltenden Olefinen, insbesondere Ethylen, mit Sauerstoff in der Dampfphase geeigneter Katalysator, umfassen eine katalytisch wirksame Menge an Silber, eine Promotormenge an Alkalimetall, eine Promotormenge an Magnesium und eine Promotormenge an Rhenium, aufgebracht auf einen Träger, der wenigstens 85 Gew.-% $\alpha$-Aluminiumoxid und von 0,001 bis 2 Gew.-% (bestimmt als Element) an Magnesium in Form eines Oxids umfaßt.

**2.** Katalysator nach Anspruch 1, worin der Träger zusätzlich von 0,01 bis 5 Gew.-% (bestimmt als Dioxid) an Silicium in Form eines Oxids und von 0,1 bis 10 Gew.-% (bestimmt als Dioxid) an Zirkonium in Form eines Oxids umfaßt.

**3.** Katalysator nach Anspruch 1, worin der Träger ein Wasserporenvolumen von 0,1 cm³/g bis 0,6 cm³/g und eine Oberfläche von 0,1 m²/g bis 3 m²/g aufweist.

**4.** Katalysator nach Anspruch 1, worin in dem Träger das Magnesiumoxid in einer Menge von etwa 0,01 Gew.-% bis etwa 0,1 Gew.-%, bezogen aus das Gesamtgewicht des Trägers, vorliegt.

**5.** Katalysator nach Anspruch 1, worin die Menge an Silber von 1 bis 25 Gew.-% des Gesamtkatalysators ausmacht, die Menge an Alkalimetallpromotor von 10 bis 1500 Teilen pro Million, ausgedrückt als Metall, bezogen auf das Gewicht des Gesamtkatalysators, betragt, die Menge an Magnesiumpromotor von 10 bis 1000 Teile pro Million, ausgedrückt als das Element, bezogen auf das Gewichts des Gesamtkatalysators, beträgt und die Menge an Rheniumpromotor von 0,1 bis 10 Mikromol Rhenium, ausgedrückt als Metall, je Gramm Gesamtkatalysator beträgt.

**6.** Katalysator nach Anspruch 1, worin das Alkalimetall, das Magnesium und das Rhenium jeweils für sich oder in einem beliebigen Gemisch auf dem Katalysator, auf dem Träger oder sowohl auf dem Katalysator als auch auf dem Träger vorliegen.

7. Katalysator nach Anspruch 1, worin der Alkalimetallpromotor unter Kalium, Rubidium, Cäsium, Lithium und Gemischen hievon ausgewählt ist.

8. Katalysator nach Anspruch 7, worin der Alkalimetallpromotor Cäsium ist.

9. Katalysator nach Anspruch 7, worin der Alkalimetallpromotor Cäsium und wenigstens ein weiteres Alkalimetall umfaßt.

10. Katalysator nach einem der Ansprüch 1-9, der zusätzlich einen unter Schwefel, Molybdän, Wolfram, Chrom und Gemischen hievon ausgewählten Rhenium-Copromotor umfaßt.

11. Katalysator nach Anspruch 1, worin der Rhenium-Copromotor unter Sulfat, Sulfit, Sulfonat, Molybdat, Wolframat, Chromat und Gemischen hievon ausgewählt ist.

12. Verfahren zur Herstellung des Katalysators nach einem der Ansprüche 1-11, welches ein Imprägnieren eines Trägers, der wenigstens 85 Gew.-% $\alpha$-Aluminiumoxid und 0,001-2 Gew.-% (bestimmt als das Element) Magnesium in Form eines Oxids umfaßt, mit einer oder mit mehreren, Lösungsmittel umfassenden Lösungen, die darin ausreichend gelöste Silberverbindung(en), Alkalimetallverbindung(en), Magnesiumverbindung(en) und Rheniumverbindung(en) enthalten, um auf dem Träger 1-25 Gew.-%, bezogen auf den Gesamtkatalysator, der Silberverbindung(en), ausgedrückt als Metall, von 10-3000 Gewichtsteile pro Million an Alkalimetallverbindung(en), ausgedrückt als das Metall und bezogen auf das Gewicht des Gesamtkatalysators, von 10-1000 Gewichtsteile pro Million Magnesiumverbindung(en), ausgedrückt als das Metall und bezogen auf das Gewicht des Gesamtkatalysators, und von 0,1 bis 10 Mikromol Rheniumverbindung(en), ausgedrückt als das Metall, je Gramm Gesamtkatalysator abzulagern, zur Ausbildung des Katalysators mit einer katalytisch wirksamen Menge an Silber, einer Promotormenge an Alkalimetall, einer Promotormenge an Magnesium und einer Promotormenge an Rhenium umfaßt.

13. Verfahren nach Anspruch 12, worin der Träger zusätzlich 0,01-5 Gew.-% (bestimmt als das Dioxid) an Silicium in Form eines Oxids und 0,1-10 Gew.-% (bestimmt als das Dioxid) an Zirkonium in Form eines Oxids umfaßt

14. Verfahren nach Anspruch 12 oder 13, worin nach dem Imprägnieren das Silber durch Erhitzen auf eine Temperatur zwischen 50°C und 600°C zu metallischem Silber reduziert wird.

15. Verfahren nach einem der Ansprüche 12-14, das zusätzlich das Imprägnieren des Trägers mit einer Lösung umfaßt, die ausreicht, um darauf 0,1-10 Mikromol je Gramm Gesamtkatalysator an Rhenium-Copromotorverbindung(en) abzulagern, ausgewählt aus der aus Schwefel, Wolfram, Molybdän, Chrom und Gemischen hievon bestehenden Gruppe, ausgedrückt als das Element, zur Ausbildung des Katalysators mit einer katalytisch wirksamen Menge an Silber, einer Promotormenge an Alkalimetall, einer Promotormenge an Magnesium, einer Promotormenge an Rhenium und einer Promotormenge an Rhenium-Copromotor.

**Revendications**

1. Catalyseur convenant à l'époxydation d'oléfines ne comportant pas d'hydrogène allylique, plus particulièrement, l'éthylène avec de l'oxygène en phase vapeur, lequel catalyseur comprend une proportion catalytique efficace d'argent, une quantité promotrice d'un métal alcalin, une quantité promotrice de magnésium, une quantité promotrice de rhénium et, éventuellement, un promoteur conjoint du rhénium choisi parmi le soufre, le molbydène, le tungstène, le chrome et leurs mélanges, supportés par un véhicule ou support comprenant au moins 85% en poids d'alpha-alumine et de 0,001 à 2% en poids (exprimés sous forme de l'élément) de magnésium sous la forme d'un oxyde.

2. Catalyseur suivant la revendication 1, caractérisé en ce que le véhicule ou support comprend, en outre, de 0,01 à 5% en poids (mesurés sous forme du dioxyde) de silicium sous la forme d'un oxyde et de 0,1 à 10% en poids (mesurés sous forme de dioxyde) de zirconium sous la forme d'un oxyde.

3. Catalyseur suivant la revendication 1, caractérisé en ce qu'il possède un volume des pores à l'eau compris entre 0,1 cc/g et 0,6 cc/g et une surface spécifique comprise entre 0,1 $m^2$/g et 3 $m^2$/g.

4. Catalyseur suivant la revendication 1, caractérisé en ce que, dans le véhicule ou support, l'oxyde de magnésium est présent en une proportion qui varie d'environ 0,01% en poids à environ 0,1% en poids, sur base du poids total du véhicule ou support.

5. Catalyseur suivant la revendication 1, caractérisé en ce que la proportion d'argent varie de 1 à 25% en poids du catalyseur total, la proportion de métal alcalin promoteur varie de 10 à 1500 parties par million, exprimées sous forme du métal, en poids, par rapport au catalyseur total, la proportion de magnésium promoteur fluctue de 10 à 1000 parties par million, exprimées sous forme de l'élément, en poids, par rapport au catalyseur total et la proportion du rhénium promoteur varie de 0,1 à 10 micromoles de rhénium, exprimées sous forme du métal, par gramme de catalyseur total.

6. Catalyseur suivant la revendication 1, caractérisé en ce que le métal alcalin, le magnésium et le rhénium se trouvent individuellement ou en mélange sur le catalyseur, sur le support, ou sur à la fois le catalyseur et le support.

7. Catalyseur suivant la revendication 1, caractérisé en ce que le métal alcalin promoteur est choisi parmi le potassium, le rubidium, le césium, le lithium et leurs mélanges.

8. Catalyseur suivant la revendication 7, caractérisé en ce que le métal alcalin promoteur est le césium.

9. Catalyseur suivant la revendication 7, caractérisé en ce que le métal alcalin promoteur comprend du césium plus au moins un métal alcalin supplémentaire.

10. Catalyseur suivant l'une quelconque des revendications 1 à 9, caractérisé en ce qu'il comprend, en outre, un promoteur conjoint du rhénium, choisi parmi le soufre, le molybdène, le tungstène, le chrome et leurs mélanges.

11. Catalyseur suivant la revendication 1, caractérisé en ce que le promoteur conjoint du rhénium est choisi parmi les sulfates, sulfites, sulfonates, molybdates, tungstates, chromates et leurs mélanges.

12. Procédé de préparation du catalyseur suivant l'une quelconque des revendications 1 à 11, caractérisé en ce que l'on imprègne un véhicule ou support comprenant au moins 85% en poids d'alpha-alumine et de 0,001 à 2% en poids (mesurés sous forme de l'élément) de magnésium, sous forme d'un oxyde, d'une ou plusieurs solutions comprenant un solvant dans lequel sont dissous un ou plusieurs composés d'argent, un ou plusieurs composés de métaux alcalins, un ou plusieurs composés de magnésium et un ou plusieurs composés de rhénium, en proportion qui suffit à déposer sur le support de 1 à 25% en poids par rapport au catalyseur total, du ou des composés d'argent, exprimés sous forme du métal, de 10 à 3000 parties par million en poids du ou des composés de métaux alcalins, exprimées sous forme du métal, en poids, par rapport au catalyseur total, de 10 à 1000 parties par million en poids du catalyseur total de composés du magnésium, exprimées sous forme de l'élément, et de 0,1 à 10 micromoles par gramme du catalyseur total du ou des composés du rhénium, exprimées sous forme du métal, pour donner au catalyseur une proportion catalytiquement efficace d'argent, une proportion promotrice de métal alcalin, une proportion promotrice de magnésium et une proportion promotrice de rhénium.

13. Procédé suivant la revendication 12, caractérisé en ce que le véhicule ou support comprend, en outre, de 0,01 à 5% en poids (mesurés sous forme de dioxyde) de silicium sous la forme d'un oxyde et de 0,1 à 10% en poids (mesurés sous forme de dioxyde) de zirconium sous la forme d'un oxyde.

14. Procédé suivant la revendication 12 ou 13, caractérisé en ce que, après l'imprégnation, l'argent est réduit en argent métallique par chauffage à une température comprise entre 50°C et 600°C.

15. Procédé suivant l'une quelconque des revendications 12 à 14, caractérisé en ce qu'il comprend, en outre, l'imprégnation du véhicule ou support d'une solution qui suffit à déposer sur ce véhicule ou support de 0,1 à 10 micromoles par gramme de catalyseur total d'un ou plusieurs composés de promoteur conjoint du rhénium, choisis dans le groupe formé par le soufre, le tungstène, le molybdène, le chrome et leurs mélanges, exprimées sous forme de l'élément, pour donner au catalyseur une proportion catalytiquement efficace d'argent, une proportion promotrice de métal alcalin, une proportion promotrice de magnésium, une proportion promotrice de rhénium et une proportion promotrice de promoteur conjoint du rhénium.